# EUROPEAN PATENT SPECIFICATION

(11) **EP 1 750 777 B1**
(45) Date of publication and mention of the grant of the patent: **14.09.2011**
(21) Application number: 05737804.4
(22) Date of filing: 04.05.2005
(51) Int. Cl.: A61L 9/14, B05B 7/28, A61L 9/12

(54) **ESSENTIAL OIL ATOMISER**
ZERSTÄUBER FÜR EIN ESSENTIELLES ÖL
ATOMISEUR D'HUILES ESSENTIELLES

(30) Priority: 04.05.2004 AU 2004902366
(43) Date of publication of application: 14.02.2007
(73) Proprietor: Air Aroma Research Pty. Limited, Sandringham, VIC 3191 (AU)
(72) Inventor: VAN ROEMBURG, John, Brighton, Victoria 3186 (AU)
(74) Representative: Alexander, Thomas Bruce
(86) International application number: PCT/AU2005/000639
(87) International publication number: WO 2005/105163

(56) References cited:
- WO-A-02/26276
- WO-A1-03/008001
- FR-A- 2 448 930
- US-A- 4 836 452
- US-A- 4 938 416
- US-A- 5 248 448
- US-A- 5 549 247
- US-A- 5 635 132
- US-A- 6 027 030
- US-A1- 2005 077 383
- US-B1- 6 238 646
- US-B2- 6 645 436

## Description

### Area of the Invention

This invention relates to the area of equipment for use with oils both fragrant and essential and in particular to an atomiser which is able to be used to spray these oils into the air.

### Background to the Invention

While the invention relates to an atomiser for use with both essential and fragrant oils it will be discussed here for convenience's sake in terms of its application to essential oil dispersion.

Essential oil dispersion in air has become a popular method of eliminating unwanted odours in the ambient environment and opposing air borne microbes or bacteria, viruses and moulds among other things. Among the other attributes of essential oil dissemination in the atmosphere is that it is believed to promote a healthy pleasant and positive environment.

It is believed that each essential oil has its own unique properties that are therapeutic in the human body in a holistic way and that they provide benefit both physically by inhaling droplets and psychologically via the sense of smell. The theory is that the aroma of an essential oil can induce a number of intricate responses including sensory stimulation, relaxation and a sense of well being.

Many essential oil dispersion means involve heating the oil until it vaporises and diffuses through a room. A problem with this approach is the fact that open flames can be a fire hazard and also it is considered by many that cold air diffusion is the only method which preserves the full integrity of the properties of the essential oil.

Essential oil dispersion means not involving heating have been proposed. US 6,645,436 discloses an essential oil diffuser which makes use of a diffuser jet, an oil reservoir, a glass top and an electric air pump. The electric air pump draws oil from the reservoir via a jet into the glass top where baffles or tongues are provided. The baffles or tongues present in the glass top separate and return to the oil reservoir essential oil vapour that is not fine enough, and so the apparatus does suffer from the problem that the desired level of micromist is not achieved.

There remains, therefore, a need for improved means to disperse essential and fragrant oils.

### Outline of the Invention

It is an object of this invention to provide a means whereby essential and fragrant oils are able to be disseminated in droplet form without the application of heat to the oil, and with improved dispersion.

The present invention provides an oil atomiser system for essential and fragrant oils comprising at least one atomiser which is adapted to engage in use with a container of said oil, said atomiser having a body with a hollow interior and having a mixer housing associated with one surface thereof, from which mixer housing an oil feeder tube extends through a surface of the body to, in use, the container of said oil, the mixer housing having also a compressed air inlet with a first diameter which is larger than a second diameter of the inlet which passes to a diffusion chamber in the mixer housing having a passage to the body of the atomiser such that, in use, application of compressed air to the inlet causes the oil to be broken down into small droplets in the diffusion chamber which then pass through the aperture and are dispersed to air in fine droplet form through at least one oil mist outlet in the body.

It is further preferred that the body have a vertical height which is such that a single plume of oil droplets is formed as an air/oil mix exits the mixer housing to the atomiser body.

It is preferred that the compressed air be provided by an air compressor associated with the system.

It is further preferred that the application of the compressed air cause essential oil to be drawn into the mixer housing and passed through a diffusion chamber to leave the system as an essential oil/air micromist mixture.

It is preferred also that a plurality of atomisers may be provided in a given housing. It is further preferred that a plurality of atomisers in a housing may be programmed to function at different times of day either by timing means or programmable means.

In order that the invention may be more readily understood we will describe by way of non limiting example a specific embodiment of the invention.

### Brief Description of the Drawing Figures

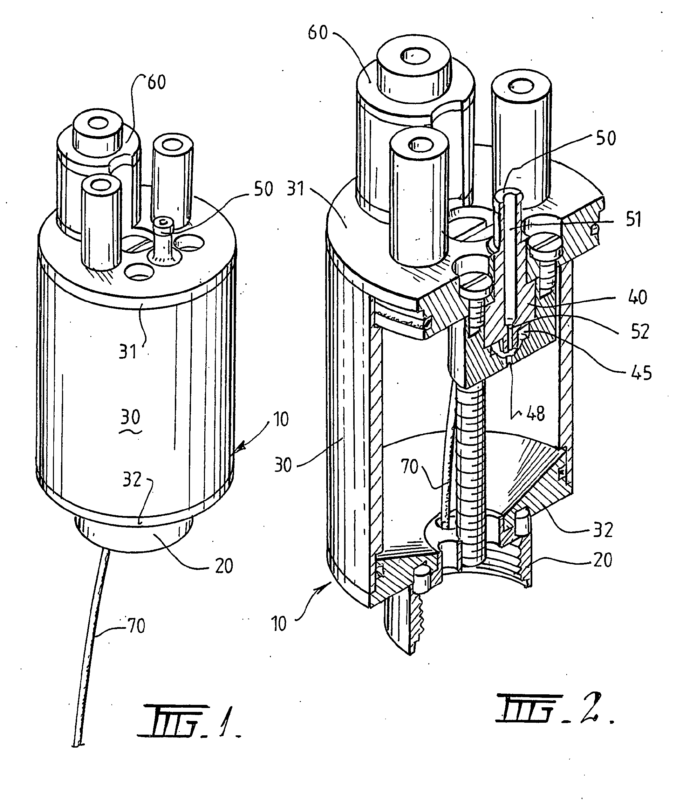

Fig. 1 Shows a perspective view of the atomiser of the invention;
Fig, 2 Shows a sectional view through the atomiser;

### Description of an Embodiment of the invention

Although the concept of the invention can applied to many different styles of essential or fragrant oil atomiser a particular embodiment of the atomiser system will be described here in terms of its application to essential oil dispersion. It is of course to be understood that variations in physical design, manufacturing materials and size can still lie within the scope of the invention as set forth in the appended claims.

In this particular embodiment of the invention an essential oil atomiser 10 is provided which is provided with engagement means, in this case a screw threaded portion 20 of body portion 30, to engage with the neck of an open bottle of essential oil.

Above this engagement means the atomiser system is provided with a generally cylindrical body 30 for its component parts which may be of stainless steel or any other preferred material. The interior of this housing body 30 holds a mixer housing 40 having a diffusion chamber 45.

The arrangement is such that an external compressed air supply passes through an air supply jet 50 into the mixer housing 40 thereby causing essential oil to be drawn through a tube 70 passing from the essential oil bottle and terminating in the mixer housing 40.

The compressed air inlet 50 has an initially wider bore 51, the air then passes through a smaller bore 52 to the diffusion chamber 45 where the oil is broken down into small droplets which pass through a narrow aperture 48 into the interior of the atomiser body 30.

The resultant mixture then passes through at least one oil mist outlet 60 in an upper surface of the atomiser body 30 to be dispersed as an essential oil/air mixture as a micromist. This ability to successfully break an essential oil up into a fine droplet distribution which is capable of remaining airborne is achieved without the use of heat to either vaporise the oil or vaporise water droplets carrying the oil.

The effect is achieved by the compressed air supply passing from the initial air supply jet into a finer tube at a terminal end of the first jet or tube. By this means the resulting jet enhances the essential oil atomisation into small droplets capable of being effectively dispersed to the environmental air.

In this embodiment of the invention the atomiser body 30 includes a top cap 31 and a bottom cap 32 which engage with its central body 30 in sealing engagement with the provision of appropriate O rings. The atomiser body 30 is maintained in its assembled configuration by screw means.

While this is the arrangement preferred in this embodiment of the invention it is envisaged that any interconnection means of the component parts could be used and that the actual system housing components could vary both in their arrangement and in their appearance within the scope of the invention set forth in the appended claims. The fundamental means whereby the essential oil was able to be atomised and dispersed would of course be common to all models of the atomiser in accordance with the scope of the invention set forth in the appended claims.

While we have described herein one specific embodiment of the invention it is envisaged that other embodiments of the invention will exhibit any number of and any combination of the features of those previously described and it is to be understood that variations and modifications in this can be made without departing from the scope of the invention as set forth in the appended claims.

## Claims

1. An oil atomiser system for essential and fragrant oils comprising at least one atomiser (10) which is adapted to engage in use with a container of said oil, said atomiser (10) having a body (30) with a hollow interior and having a mixer housing (40) associated with one surface thereof, from which mixer housing (40) an oil feeder tube (70) extends through a surface of the body (30) to, in use, the container of said oil, the mixer housing (40) having also a compressed air inlet (50) with a first diameter (51) which is larger than a second diameter (52) of the inlet (50) which passes to a diffusion chamber (45) in the mixer housing (40) having a passage (48) to the body (30) of the atomiser (10) such that, in use, application of compressed air to the inlet (50) causes the oil to be broken down into small droplets in the diffusion chamber 45 which then pass through the aperture (48) and are dispersed to air in fine droplet form through at least one oil mist outlet (60) in the body (30).

2. An oil atomiser system as claimed in claim 1 wherein the mixer housing (40) is located inside the body and is integral with an upper surface thereof.

3. An oil atomiser system as claimed in claim 1 or claim 2 wherein the application of the compressed air causes oil to be drawn into the mixer housing (40) and passed through the diffusion chamber (45) and through the aperture (48) to the atomiser body (30) which is dimensioned such that an oil/air micromist mixture passes to the atomiser body (30).

4. An oil atomiser system as claimed in claim 3 wherein the atomiser body (30) has a vertical height such that a single plume of oil droplets is formed as the air/oil micromist mixture exits the mixer housing (40) to the atomiser body (30).

5. An oil atomiser system as claimed in claim 4 wherein the compressed air is provided by an air compressor associated with the atomiser (10).

6. An oil atomiser system as claimed in claim 5 wherein a plurality of atomisers (10) are provided in a housing.

7. An oil atomiser system as claimed in claim 6 wherein each atomiser (10) in the housing is programmed to function at a different time of day by timing means.

8. An oil atomiser system as claimed in claim 7 wherein operation of individual atomisers (10) in the housing is programmable.

9. An oil atomiser system as claimed in any one of claims 1 to 8 wherein the atomiser (10) engages in use with the container of oil by engagement means (20).

## Patentansprüche

1. Öl-Zerstäuber-System für ätherische und duftende Öle, umfassend wenigstens einen Zerstäuber (10), der angepasst ist, um im Betrieb mit einem Behälter für das Öl in Eingriff zu stehen, wobei der Zerstäuber (10) einen Körper (30) mit einem hohlen Inneren aufweist und ein Mischer-Gehäuse (40) aufweist, das einer Fläche davon zugeordnet ist, wobei ein Öl-Zufuhr-Rohr (70) sich von dem Mischer-Gehäuse (40) durch eine Fläche des Körpers (30) zu, im Betrieb, dem Behälter für das Öl erstreckt, wobei das Mischer-Gehäuse (40) weiter einen Einlass (50) für komprimierte Luft mit einem ersten Durchmesser (51) aufweist, der größter ist als ein zweiter Durchmesser (52) des Einlasses (50), der zu einer Diffusionskammer (45) in dem Mischer-Gehäuse (40) führt, die einen Durchgang (48) zu dem Körper (30) des Zerstäubers (10) aufweist, so dass, im Betrieb, eine Beaufschlagung des Einlasses (50) mit komprimierter Luft bewirkt, dass das Öl in kleine Tröpfchen in der Diffusionskammer (45) aufgebrochen wird, die dann durch die Öffnung (48) hindurch treten und durch wenigstens einen Ölnebel-Auslass (60) in dem Körper (30) in der Form feiner Tröpfchen in der Luft verteilt werden.

2. Öl-Zerstäuber-System nach Anspruch 1, wobei das Mischer-Gehäuse (40) sich innerhalb des Körpers befindet und integral mit einer oberen Fläche davon ausgebildet ist.

3. Öl-Zerstäuber-System nach Anspruch 1 oder Anspruch 2, wobei die Beaufschlagung mit der komprimierten Luft bewirkt, dass Öl in das Mischer-Gehäuse (40) eingesogen und durch die Diffusionskammer (45) geleitet wird, und durch die Öffnung (48) zu dem Zerstäuber-Körper (30), wobei die Dimensionierung so ist, dass ein Öl/Luft-Mikronebel-Gemisch zu dem Zerstäuber-Körper (30) geleitet wirt.

4. Öl-Zerstäuber-System nach Anspruch 3, wobei der Zerstäuber-Körper (30) eine vertikale Höhe hat, so dass eine einzelne Wolke von ÖlTröpfchen gebildet wird, wenn das Luft/Öl-Mikronebel-Gemisch das Mischer-Gehäuse (40) zu dem Zerstäuber-Körper (30) hin verlässt.

5. Öl-Zerstäuber-System nach Anspruch 4, wobei die komprimierte Luft von einem Luft-Kompressor bereitgestellt wird, der dem Zerstäuber (10) zugeordnet ist.

6. Öl-Zerstäuber-System nach Anspruch 5, wobei eine Mehrzahl von Zerstäubern (10) in einem Gehäuse vorgesehen sind.

7. Öl-Zerstäuber-System nach Anspruch 6, wobei jeder Zerstäuber (10) in dem Gehäuse programmiert ist, zu einer unterschiedlichen Tageszeit zu arbeiten, durch Zeitgebermittel.

8. Öl-Zerstäuber-System nach Anspruch 7, wobei ein Betrieb von einzelnen Zerstäubern (10) in dem Gehäuse programmierbar ist.

9. Öl-Zerstäuber-System nach einem der Ansprüche 1 bis 8, wobei der Zerstäuber (10) im Betrieb mit dem Behälter für Öl vermittels Eingriffsmitteln (20) in Eingriff steht.

## Revendications

1. Système d'atomiseur d'huile pour des huiles essentielles et parfumées comprenant au moins un atomiseur (10) qui est adapté pour s'engager en utilisation avec un flacon de ladite huile, ledit atomiseur (10) ayant un corps (30) avec un intérieur creux et ayant un boîtier mélangeur (40) associé à l'une de ses surfaces, boîtier mélangeur (40) à partir duquel un tuyau (70) d'alimentation en huile s'étend à travers une surface du corps (30), en cours d'utilisation, jusqu'au flacon de ladite huile, le boîtier mélangeur (40) ayant également une entrée (50) d'air comprimé avec un premier diamètre (51) qui est plus important qu'un deuxième diamètre (52) de l'entrée (50) qui passe dans une chambre (45) de diffusion dans le boîtier mélangeur (40) ayant un passage (48) jusqu'au corps (30) de l'atomiseur (10) de sorte qu'en cours d'utilisation, l'application d'air comprimé à l'entrée (50) amène l'huile à se désintégrer en petites gouttelettes dans la chambre (45) de diffusion qui passent ensuite à travers l'ouverture (48) et sont dispersées dans l'air sous forme de fines gouttelettes à travers au moins une sortie (60) de nuage d'huile dans le corps (30).

2. Système d'atomiseur d'huile tel que revendiqué dans la revendication 1, dans lequel le boîtier mélangeur (40) est situé à l'intérieur du corps et est solidaire de sa surface supérieure.

3. Système d'atomiseur d'huile tel que revendiqué dans la revendication 1 ou la revendication 2, dans lequel l'application de l'air comprimé amène l'huile à se désintégrer dans le boîtier mélangeur (40) et passe à travers la chambre (45) de diffusion et à travers l'ouverture (48) au corps (30) de l'atomiseur qui est dimensionné de sorte qu'un mélange de micro-nuage huile/air passe au corps (30) de l'atomiseur.

4. Système d'atomiseur d'huile tel que revendiqué dans la revendication 3, dans lequel le corps (30) de l'atomiseur a une hauteur verticale de sorte qu'un seul panache de gouttelettes d'huile soit formé à mesure que le mélange de micro-nuage huile/air quitte le boîtier mélangeur (40) vers le corps (30) de l'atomiseur.

5. Système d'atomiseur d'huile tel que revendiqué dans la revendication 4, dans lequel l'air comprimé est fourni par un compresseur d'air associé à l'atomiseur (10).

6. Système d'atomiseur d'huile tel que revendiqué dans la revendication 5, dans lequel une pluralité d'atomiseurs (10) sont prévus dans un boîtier.

7. Système d'atomiseur d'huile tel que revendiqué dans la revendication 6, dans lequel chaque atomiseur (10) dans le boîtier est programmé pour fonctionner à un autre moment de la journée par un moyen de synchronisation.

8. Système d'atomiseur d'huile tel que revendiqué dans la revendication 7, dans lequel le fonctionnement d'atomiseurs individuels (10) dans le boîtier est programmable.

9. Système d'atomiseur d'huile tel que revendiqué dans l'une quelconque des revendications 1 à 8, dans lequel l'atomiseur (10) s'engage une utilisation avec le flacon d'huile par un moyen (20) d'engagement.
